# EUROPEAN PATENT SPECIFICATION

(11) **EP 4 593 937 B1**
(45) Date of publication and mention of the grant of the patent: **10.06.2026**
(21) Application number: 23783037.7
(22) Date of filing: 19.09.2023
(51) Int. Cl.: A61N 1/05, A61N 1/372, A61N 1/375

(54) **MULTI-ELECTRODE IMPLANTABLE MEDICAL DEVICE**
IMPLANTIERBARES MEDIZINISCHES GERÄT MIT MEHREREN ELEKTRODEN
DISPOSITIF MÉDICAL IMPLANTABLE À ÉLECTRODES MULTIPLES

(30) Priority: 29.09.2022 US 202263377625 P
(43) Date of publication of application: 06.08.2025
(73) Proprietor: Medtronic, Inc., Minneapolis, Minnesota 55432 (US)
(72) Inventor: GRUBAC, Vladimir, Minneapolis, Minnesota 55432 (US); WINANS, Allison E., Minneapolis, Minnesota 55432 (US); KIRSCH, Lydia C., Minneapolis, Minnesota 55432 (US)
(74) Representative: Zimmermann & Partner Patentanwälte mbB
(86) International application number: PCT/IB2023/059279
(87) International publication number: WO 2024/069314

(56) References cited:
- WO-A1-2022/173646
- US-A1- 2015 045 868
- US-A1- 2020 197 706
- US-A1- 2021 046 306
- US-A1- 2021 154 477
- US-A1- 2021 275 824
- US-A1- 2022 257 933
- US-A1- 2022 265 997

## Description

### TECHNICAL FIELD

This disclosure is related to medical device systems, such as relatively compact implantable medical devices and associated fixation components.

### BACKGROUND

In some examples, implantable cardiac pacemakers include a pulse generator device to which one or more flexible elongate lead wires are coupled. The pulse generator device may be implanted in a subcutaneous pocket, remote from the heart, and each of the one or more lead wires extends therefrom to a corresponding electrode, coupled thereto and positioned at a pacing site, either endocardial or epicardial. Mechanical and/or MRI compatibility issues may be associated with elongate lead wires. Relatively compact implantable medical devices (IMDs) have been developed that are wholly contained within a relatively compact package, the entirety of which is configured for implant in close proximity to the pacing site, e.g., within a chamber of the heart. US 2022/257933 A1, WO 2022/173646 A1, and US 2021/046306 A1 relate to fixation components for multi-electrode implantable medical device.

### SUMMARY

This disclosure describes IMDs that include a fixation component with a penetrator tine and an electrode interface assembly. The penetrator tine of the fixation component may operate in conjunction with one or more elongate electrodes (e.g., a leadlet) mounted in proximity to a distal end of an IMD. For example, the penetrator tine may define an exoskeleton of a cardiac pacing electrode. The penetrator tine may operate in conjunction with an electrode interface assembly configured to control a depth of tissue penetration of the penetrator tine. When deployed, the penetrator tine and electrode interface assembly may provide improved tissue fixation, controlled penetration to a selected depth within a tissue, improved electrode tissue interface, and/or improved tissue disengagement compared to fixation components without the described penetrator tine and/or electrode interface assembly. In this way, the fixation component may facilitate implanting and/or extracting IMDs.

In some examples, this disclosure describes an implantable medical device (IMD) including: a device body extending from a proximal portion to a distal portion along a longitudinal axis; a fixation component including a penetrator tine extending away from the distal portion of the device body and configured to penetrate a tissue; and an electrode interface assembly including: a leadlet extending away from the distal portion of the device body; an elongated body extending away from the distal portion of the device body, the elongated body defining a recess and a groove, wherein the groove is configured to receive the leadlet; and an electrode extending from the elongated body and disposed within the recess, wherein the electrode is configured to form an electrical circuit with the tissue, and wherein the elongated body is configured to place the electrode in contact with the tissue.

In some examples, this disclosure describes an IMD including a device body extending from a proximal portion to a distal portion along a longitudinal axis; a fixation component including: a penetrator tine extending away from the distal portion of the device body and configured to penetrate a tissue; an electrode interface assembly including: an elongated body extending from the distal portion of the device body, the elongated body defining a first recess and a second recess; a first electrode extending away from the electrode interface assembly, the first electrode being disposed within the first recess; and a second electrode extending away from the electrode interface assembly, the second electrode being disposed within the second recess, wherein the first electrode is configured to form a first electrical circuit with the tissue, wherein the second electrode is configured to form a second electrical circuit with the tissue, and wherein the elongated body is configured to place the first electrode and the second electrode in contact with the tissue.

In some examples, this disclosure describes a medical device system including: an IMD including: a device body extending from a proximal portion to a distal portion along a longitudinal axis; a leadlet extending away from the distal portion of the device body, the leadlet including a first electrode disposed on a distal end of the leadlet; a fixation component including a penetrator tine extending from the distal portion of the device body and configured to penetrate a tissue; an elongated body extending from the distal portion of the device body, the elongated body defining a groove configured to receive the leadlet; a second electrode disposed with a recess of the elongated body, wherein the elongated body is configured to place the second electrode in contact with the tissue; and a delivery tool including a tubular sidewall that defines a lumen into which the IMD may be loaded, wherein the lumen has a distal opening through which the IMD may be deployed.

In some examples, this disclosure describes a method of forming an IMD, the method including: forming a device body of the IMD, wherein the device body extends from a proximal portion to a distal portion along a longitudinal axis; attaching an electrode interface assembly to the distal portion of the device body, wherein the electrode interface assembly includes: a leadlet extending away from the distal portion of the device body of the IMD; an elongated body extending away from the distal portion of the device body, the elongated body defining a groove configured to receive the leadlet; and an electrode extending away from the distal portion of the device body and disposed within a recess in the elongated body, wherein the elongated body is configured to place the electrode in contact with the tissue; and attaching a fixation component to the distal portion of the device body, wherein the fixation component includes a penetrator tine extending from the distal portion of the device body and configured to penetrate a tissue.

The details of one or more examples are set forth in the accompanying drawings and the description below. Other features, objects, and advantages will be apparent from the description and drawings, and from the claims.

### BRIEF DESCRIPTION OF DRAWINGS

FIG. 1 is a conceptual diagram illustrating a portion of an example medical device system configured to implant an implantable medical device at a target implant site.
FIGS. 2A-2B are conceptual diagrams illustrating an IMD including a fixation component and an electrode interface assembly.
FIG. 3 is a conceptual diagram illustrating an exploded view of the example IMD of FIGS. 2A-2B.
FIG. 4 is a conceptual diagram illustrating an electrode interface assembly of FIGS. 2A-2B.
FIG. 5 is a conceptual diagram illustrating the example IMD of FIGS. 2A-2B implanted at the target implant site.
FIG. 6 is a conceptual diagram illustrating a partial cut-away section of an example medical device system including a delivery tool and an IMD.
FIG. 7 is a flow diagram illustrating an example method of manufacturing an example IMD.

### DETAILED DESCRIPTION

This disclosure describes implantable medical devices (IMDs), components of IMDs, medical device systems including IMDs, and related techniques of using and forming IMDs. An example IMD may include a body, one or more elongate leadlets, a fixation component that includes a base and a plurality of tines, and an electrode interface assembly (EIA). One or more tines of the fixation component can be configured to penetrate a tissue, and the EIA can be configured to control a depth of tissue penetration of the one or more tines.

In some examples, the body of the IMD may extend along a longitudinal axis from a proximal portion of the body to a distal portion, and may enclose components of the IMD, such as circuitry and a battery. The body of the IMD may support a return electrode configured to allow electrical energy to flow from the tissue to the IMD through the return electrode.

A proximal end of an EIA may be mounted to the body, e.g., in proximity to the distal end of the body. The EIA may include one or more, or all of, the electrodes of the IMD and at least one of the electrodes of the IMD may be mounted in the EIA, e.g., within a corresponding recess within the EIA. In some examples, the EIA defines a cannula configured to retain a leadlet extending from the distal end of the body. A distal end of the leadlet may include an electrode, such as one electrode of the one or more electrodes of the IMD. The electrodes of the IMD may be configured to sense electrical signals from tissue and/or deliver electrical therapies to the tissue.

The fixation component of the IMD may include a base and a plurality of tines. The base may define a longitudinal axis of the fixation component, and a proximal end and a distal end of the IMD may be aligned along the longitudinal axis. The longitudinal axis of the fixation component and IMD may be parallel and, in some cases, the fixation component and IMD may share a longitudinal axis. The base may be fixedly attached to the IMD near the distal end of the IMD. The plurality of tines includes a penetrator tine, and may include additional tines, such as support tines, deployment tines, alignment tines, or protector tines. The plurality of tines may be spaced apart from one another around a perimeter of the distal end of the IMD and extend from the base. A shape of each respective tine of the plurality of tines may be selected to control deployment of the IMD, tissue fixation, and/or tissue disengagement. For example, the shape of a respective tine may include a number of preset curves on the respective tine, a curvature (e.g., radius) of each preset curve on the respective tine, a length of each preset curve, a length of optional straight sections between preset curves, a width of the respective tine or sections thereof (e.g., one or more tapered portions), a thickness of the respective tine, a number of cutouts along the length of the respective tine, shapes of cutouts, or any combination thereof.

The IMD includes an electrode interface assembly (EIA) that may operate in conjunction with the fixation component to control a depth of tissue penetration of one or more tines. For example, the EIA may be configured to contact the tissue, and thus resist further tissue penetration, to control the depth of tissue penetration of the penetrator tine. In some examples, the EIA includes a texture around an outer surface of the EIA, e.g., to control the depth of tissue penetration of the penetrator tine and/or prevent dislodgement of the one or more tines from the tissue. The EIA may include a proximal section attached to and extending distally from the distal portion of the body of the IMD along the longitudinal axis. The EIA may further include a distal section extending from the proximal section of the EIA and defining a non-incisive distal end. The distal section of the EIA may be configured to contact the tissue to control a depth of tissue penetration of the penetrator tine. For example, the EIA may be formed (e.g., dimensioned) to have a proximal section and distal section with a total length that results in contact between the EIA and the tissue at a selected depth of tissue penetration of the penetrator tine. Additionally, or alternatively, the EIA may be positioned on the IMD to result in contact between the EIA and the tissue at a selected depth of tissue penetration of the penetrator tine. In any case, the EIA may be configured to achieve a selected distance between the incisive distal section of the penetrator tine and the distal end of the EIA (e.g., within a range from about 4 millimeters to about 10 millimeters).

The EIA may place a first electrode of the IMD (e.g., a distal-most electrode within the EIA, such as an electrode on the distal end of the leadlet), within the tissue at a predetermined depth (e.g., at the selected depth of tissue penetration of the penetrator tine). The EIA may place a second electrode of the IMD (e.g., a proximal-most electrode disposed within a recess within the EIA) in contact with the tissue without penetrating the tissue.

When deployed, the plurality of tines, in conjunction with the EIA, may provide a selected tissue fixation. For example, the deformable preset curve of a penetrator tine may have a shape selected to penetrate tissue in a selected direction and a selected depth to fixate the IMD to the tissue. Furthermore, the distal section of the EIA may be configured to contact the tissue when the penetrator tine reaches the selected depth of tissue penetration, resisting further tissue penetration and thus controlling the depth of tissue penetration of the penetrator tine. Similarly, in another example, the deployed (e.g., undeformed) configuration of the penetrator tine, in conjunction with the EIA, may be selected to sufficiently fixate the IMD to the selected tissue.

The IMD may be loaded into a delivery catheter by deforming the deformable preset curvature of each respective tine of the plurality of tines and, optionally, a deformable portion of the EIA. When deployed at a target implant site (e.g., by allowing the tines to transition to the deployed configuration), the tines have a deployment stiffness that enables the tines to penetrate the tissue at a target implant site. By controlling the deployment stiffness, the tines may have improved tissue fixation, including control of a depth of tine penetration and an amount of tissue engagement in a lateral direction.

In some examples, the penetrator tine and the EIA may operate in conjunction with the leadlet. The penetrator tine may be configured to penetrate or cut a tissue to form a puncture in the tissue. The EIA may be configured to protect the leadlet during deployment, such as, by applying a force to the leadlet in the direction of the penetrator tine during and/or after deployment (e.g., via the inner surface of the cannula defined by the EIA), thereby reducing an undesired displacement of the leadlet or mechanical damage to the structure of the leadlet. For example, during deployment from the delivery catheter, the penetrator tine may initially penetrate a selected tissue to form a puncture, and EIA may apply a force to the first leadlet, thus urging the first leadlet toward the penetrator tine and guiding the first leadlet into the puncture. As the fixation component is further deployed, the penetrator tine may return from a deformed (e.g., pre-deployment) configuration to (or at least towards) the undeformed (e.g., deployed) configuration. As the leadlet is inserted into the puncture in the tissue, a portion of the distal portion of the EIA (e.g., the portion defining the cannula) may be inserted into the puncture in the tissue (e.g., to improve surface contact between the EIA and the tissue, to protect the leadlet, or the like). Upon reaching a selected depth of tissue penetration of the penetrator tine, a portion EIA (e.g., a portion of the EIA defining the recess for the proximal-most electrode) may contact the tissue to resist further tissue penetration.

When the penetrator tine is in the deployed configuration, the leadlet may extend in a distal direction between the penetrator tine and the EIA and from a distal end of the EIA, such that the leadlet extends from the distal end of the body of the IMD to reach the selected target tissue. In this way, the fixation component and the EIA may provide improved electrode penetration to a selected depth within the selected tissue and improved electrode contact with the selected tissue.

After deployment at the target implant site, a deflection stiffness of the penetrator tine enables a clinician to confirm adequate fixation of the penetrator tine into tissue of a patient. For example, a pull test or a tug test may be performed under fluoroscopy to confirm that the penetrator tine has engaged the tissue to confirm adequacy of implantation of the IMD. The pull test or tug test may include the clinician pulling or tugging on the deployed IMD, e.g., via a tether coupled to a proximal end of the IMD, and observing movement of the penetrator tine to determine if the penetrator tine is engaged in tissue. For example, the penetrator tine that is embedded in tissue may deflect or bend as the deployed IMD is pulled or tugged in the proximal direction. By controlling the deflection stiffness, the penetrator tine may have an improved flexibility that enables a clinician to more easily confirm tissue engagement.

In some examples, the tines may be configured to disengage from the tissue. For example, the IMD may be retrieved by a retrieval catheter. During retrieval, a retrieval member of the retrieval catheter may engage a proximal end of the IMD, such as a retrieval structure. The retrieval member may be withdrawn in a proximal direction into the retrieval catheter. Withdrawing the retrieval member in the proximal direction may cause the tines to move from the undeformed configuration to the deformed configuration as the tissue resists movement of the tines. By moving from the undeformed configuration to the deformed configuration during retrieval, the tines may improve tissue disengagement to facilitate extracting the IMD, compared to tines that do not move from an undeformed configuration to a deformed configuration during retrieval from a tissue.

In this disclosure, the example systems, devices, and techniques will be described with reference to delivering an IMD configured as a cardiac pacemaker to a target site in a heart of a patient. However, it will be understood that example systems, devices, and techniques of the present disclosure are not limited to delivering such IMDs to a target site in the heart. For example, the example systems, devices, and techniques described herein may be used to deliver other medical devices, such as drug delivery device, sensing devices, neurostimulation device, or medical electrical leads. Additionally, the example systems, devices, and techniques described herein may be used to deliver any such IMDs to other locations within a body of a patient. In short, the example systems, devices, and techniques described herein can find useful application in delivery of a wide variety of implantable medical devices for delivery of therapy to a patient or patient sensing.

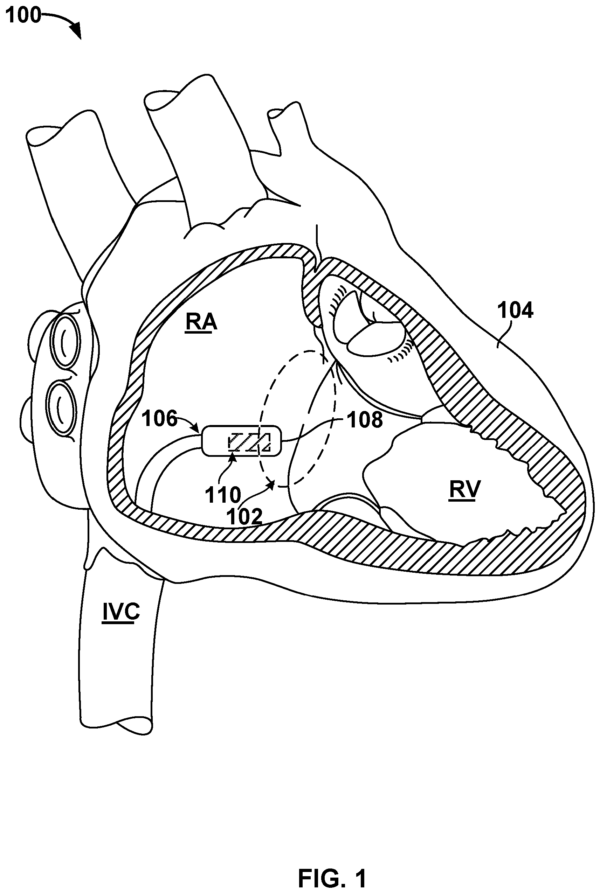

FIG. 1 is a conceptual diagram illustrating a portion of an example medical device system 100 configured to implant a relatively compact implantable medical device 110 ("IMD 110") at a target implant site 102. In some examples, as illustrated in FIG. 1, the target implant site 102 may include an appendage of a right atrium (RA) (e.g., the triangle of Koch) of the heart 104 of a patient. In some examples, target implant site 102 may include other portions of heart 104 or other locations within a body of the patient. Medical device system 100 may include a delivery tool 106 configured to house and controllably deploy relatively compact IMD 110. In some examples, a clinician may maneuver medical device system 100 to target implant site 102. For example, with the IMD loaded therein, the clinician may guide delivery tool 106 through the inferior vena cava (IVC) or superior vena cava (SVC) and into the RA. In some examples, other pathways or techniques may be used to guide delivery tool 106 into other target implant sites within the body of the patient.

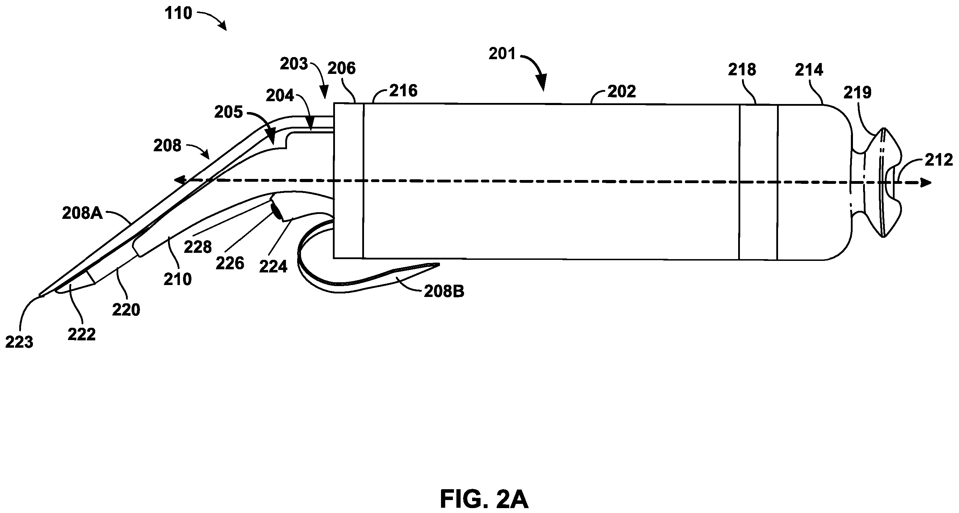

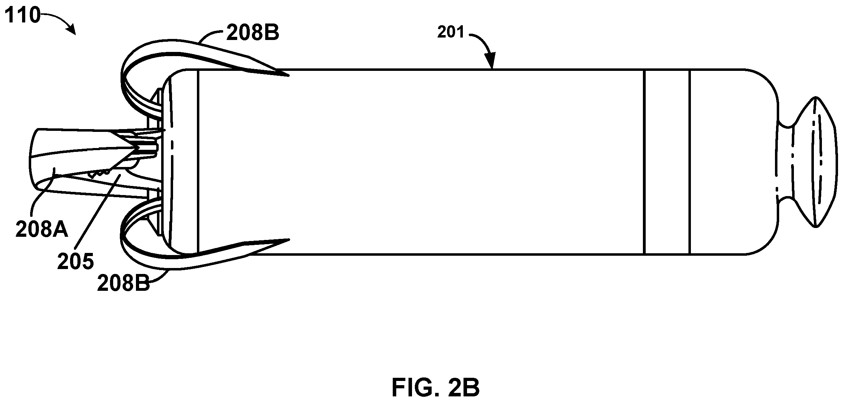

FIGS. 2A and 2B are conceptual diagrams illustrating IMD 110 including a fixation component 203 and an electrode interface assembly 204 ("EIA 204"). Fixation component 203 and EIA 204 may be connected (e.g., removably connected, permanently connected) to body 201 of IMD 110 (also referred to as "device body 201"). Fixation component 203 includes a base 206 and one or more tines 208. Body 201 is defined by housing 202 extending along longitudinal axis 212 from a proximal portion 214 to a distal portion 216. Body 201 may include an inner chamber defined by housing 202. Housing 202 may include a biocompatible and biostable metal such as titanium. In some examples, Housing 202 may form a hermetically sealed body 201. Housing 202 may include a nonconductive coating and define a return electrode 218 as an uncoated portion of housing 202. IMD 110 may include any suitable dimensions. In some examples, an outer diameter of IMD 110 (e.g., outer diameter of body 201) may be between about 10 French (Fr) and about 30 Fr, such as about 20 Fr.

As discussed above, fixation component 203 may include base 206 and one or more tines 208. Base 206 may have a proximal end and a distal end aligned along a longitudinal axis, which may or may not be longitudinal axis 212 of body 201. The proximal end of base 206 may be attached to the distal portion of body 201.

One or more tines 208 may extend from base 206. For example, as illustrated in FIG. 2A, a penetrator tine 208A may extend from base 206. Penetrator tine 208A is configured to penetrate a tissue to fix IMD 110 to the tissue. Penetrator tine 208A may be configured to hold one or more electrodes (e.g., first electrode 222 on leadlet 220) in contact with tissue at a target implant site, e.g., target implant site 102. Penetrator tine 208A may define a distal tip 223. Distal tip 223 may be configured to pierce tissue of heart 104 and form a puncture within the tissue.

In addition to penetrator tine 208A, fixation component 203 may include one or more other tines 208, such as one or more support tines 208B. Tines 208 may include one or more sections. For example, tines 208 may include an elastically deformable material preset into one or more curved sections and one or more optional substantially straight sections. In some examples, penetrator tine 208A and/or other tines may include one of a plurality of preset curvatures. In other examples, tines 208 may define a ribbon shape configured to deform along a plane normal to longitudinal axis 212 and to resist twisting outside of the plane. For example, each of tines 208 may deform within a corresponding plane within which the respective tine resides in an unstressed configuration.

Tines 208 may be configured to have a target deflection stiffness and a target deployment stiffness. The target deflection stiffness may include a measure of a resistance to force applied to IMD 110 in a proximal direction when fixation component 203 is engaged with tissue at target site 102. In some examples, the target deflection stiffness may be selected to enable tines 208 to deflect a predetermined amount to enable visualization of (e.g., the tissue engagement status of) tines 208 under fluoroscopy. In some examples, the target deflection stiffness may be within a range from about 0.2 Newtons (N) to about 0.8 N, such as about 0.3 N to about 0.6 N. The deployment stiffness may include a measure of a force applied by tines 208 as tines 208 move from a deformed configuration to an undeformed configuration upon deployment of fixation component 203 from distal opening 108 of delivery tool 106 (FIG. 1) such that the free distal end of tines 208 penetrates pectinate muscle (PM). In some examples, the target deployment stiffness may be within a range from about 0.6 N to about 1.2 N.

IMD 110 includes EIA 204 having a proximal section (not shown) that defines a proximal end and a distal section 205 that defines a non-incisive distal end. Distal section 205 may be an elongated body and may include a first elongated structure 210 and a second elongated structure 224. As illustrated in FIG. 2A, first elongated structure 210 may extend from distal portion 216 of body 201 and may define a cannula (not pictured) configured to retain leadlet 220. First elongated structure 210 may be sized to at least partially enter a puncture created by penetrator tine 208A and first electrode 222 disposed on a distal end of leadlet 220 at a selected depth within the tissue. First elongated structure 210 may taper from a proximal end towards a distal end (e.g., towards distal tip 223 of penetrator tine 208A, e.g., to facilitate insertion of first elongated structure 210 within a puncture in the tissue. The cannula defined by first elongated structure 210 may extend from distal portion 216 of body 201 to a distal end of first elongated structure 210 and may be sized to retain leadlet 220. In some examples, the cannula may be fully enclosed within first elongated structure 210. In some examples, the cannula may be partially open, e.g., to facilitate deflection of leadlet 220 towards penetrator tine 208A when IMD 110 in a pre-deployment configuration. In some examples, first elongated structure 210 may include texturing (e.g., a roughened, ridged, patterned, or non-smooth or increased-friction surface texture) disposed on an outer surface of first elongated structure 210 and at least partially along first elongated structure 210.

Second elongated structure 224 may extend from distal portion 216 of body 201 and define a recess (not pictured) configured to retain electrode 226. Second elongated structure 224 may be configured to control the depth of tissue penetration of penetrator tine 208A. For example, EIA 204 may be formed such that distal end 228 of second elongated structure 224 contacts the tissue at a selected depth of tissue penetration of penetrator tine 208A. A shape of second elongated structure 224 may be bulbous, polygonal, prismatic, or any other geometry with distal end 228 that may be used as a non-incisive distal end to contact tissue. Additionally, the size (e.g., length and/or girth) of second elongated structure 224 may be such that EIA 204 is configured to achieve a selected distance between distal tip 223 of penetrator tine 208A and distal end 228 of second elongated structure 224, thereby controlling the depth of tissue penetration of penetrator tine 208A. Similarly, second elongated structure 224 may be positioned on IMD 110 to achieve the selected distance between distal tip 223 of penetrator tine 208A and distal end 228. Second elongated structure 224 may be configured to control the depth of tissue penetration of penetrator tine 208A by contacting the tissue and resisting further penetration by penetrator tine 208A. In some examples, first elongated structure 210 and second elongated structure 224 may form into single elongated body 205 in a single process. In some examples, first elongated structure 210 and second elongated structure 224 may be formed separately and assembled to form single elongated body 205.

IMD 110 may contain electronic circuitry, including one or more of sensing circuitry (e.g., for sensing cardiac signals), therapy delivery circuitry (e.g., for generating cardiac pacing pulses), and processing circuitry for controlling the functionality of IMD 110, and may include leadlet 220. Leadlet 220 may terminate in a first electrode 222. In some examples, first electrode 222 may be connected to electronic circuitry without use of leadlet 220.

Leadlet 220 may include a feedthrough assembly connected to the electronic circuitry. The feedthrough assembly may include a conductor, such as an electrically conductive material, extending through a non-conductive jacket, such as polytetrafluoroethylene (PTFE) coating or polyether ether ketone (PEEK) tube, a portion of the conductor being exposed at first electrode 222. To avoid altering the properties of one or more tines 208 (e.g., penetrator tine 208A) made of a material (e.g., nitinol) that can be affected by electricity, the non-conductive jacket may extend past the one or more tines 208. The electronic circuitry may be configured to generate and deliver an electrical pulse therapy to tissue proximate to leadlet 220 via first electrode 222, through the tissue to return electrode 218. Leadlet 220 may be spaced apart from distal portion 216 of body 201, for example, being coupled to the sensing and therapy delivery circuitry within body 201 by the conductor(s) of a hermetic feedthrough assembly (not shown in FIG. 2A).

IMD 110 may also include a second electrode 226. Second electrode 226 may extend from distal portion 216 of body 201 to distal end 228 of second elongated structure 224 via a recess defined by second elongated structure 224. Second electrode 226 may be similar to first electrode 222 in at least some aspects. For example, second electrode 226 may be connected to electronic circuitry within body 201 via a corresponding feedthrough assembly. The feedthrough assembly may include a conductor, such as an electrically conductive material, extending through a non-conductive jacket, such as PTFE coating or a PEEK tube, a portion of the conductor being exposed at second electrode 226. First electrode 222 may function in conjunction with second electrode 226 for bipolar pacing and sensing, and/or first electrode 222 and second electrode 226 may each separately function with return electrode 218 for pacing and sensing of different selected tissues. In some examples, body 201 may be overlaid with an insulative layer, for example, medical grade polyurethane, parylene, or silicone. In some examples, the insulative layer may define return electrode 218 and/or second electrode 226, for example, by removing a portion of the insulative layer to expose the metallic surface of housing 202. Second electrode 226 may be used with first electrode 222 for pacing and/or sensing.

As described above, each of first electrode 222 and second electrode 226 may be connected to the electronic circuitry via a corresponding feedthrough assembly. The feedthrough assemblies may terminate into a pad, e.g., to connect to a spring connector connected to the electronic circuitry. In other examples, each feedthrough assembly may terminate into a separate wire or other conductor(s) which may be connected (e.g., welded) to the electronic circuitry. The corresponding feedthrough assemblies may be electrically isolated from each other.

First elongated structure 210 and second elongated structure 224 may be configured to place the feedthrough assemblies of the electrodes (e.g., first electrode 222, second electrode 226) in a particular arrangement. The feedthrough assemblies may be parallel to each other and to longitudinal axis 212. The feedthrough assemblies may be placed on a same reference plane (e.g., a reference plane intersecting longitudinal axis 212, a reference plane parallel to longitudinal axis 212, or the like). In some examples, one feedthrough assembly may be disposed along longitudinal axis 212 and another feedthrough assembly may be parallel to and offset from the longitudinal axis 212 by a determined distance. In some examples, each of the feedthrough assemblies may be separated from one or more other feedthrough assemblies and/or longitudinal axis 212 by a predetermined distance.

EIA 204 may be removably or permanently connected to body 201. EIA 204 may be pinned, welded, or locked to body 201 (e.g., via a thread-locking mechanisms, via a mechanical-locking mechanisms, or the like). For example, the proximal portion of EIA 204 may be disposed within body 201 and be pinned to housing 202 via one or more pins. In another example, the proximal portion of EIA 204 may be welded to a ring and/or cap of body 201.

In some examples, IMD 200 may include a retrieval structure 219 fixedly attached to proximal portion 214 of body 201. Retrieval structure 219 may be configured for temporarily tethering IMD 110 to a delivery catheter or a retrieval catheter, such as delivery tool 106. In some examples, retrieval structure 219 may be configured to couple to tether assemblies, such as those described in U.S. Patent No. 11,331,475, entitled "TETHER ASSEMBLIES FOR MEDICAL DEVICE DELIVERY SYSTEMS".

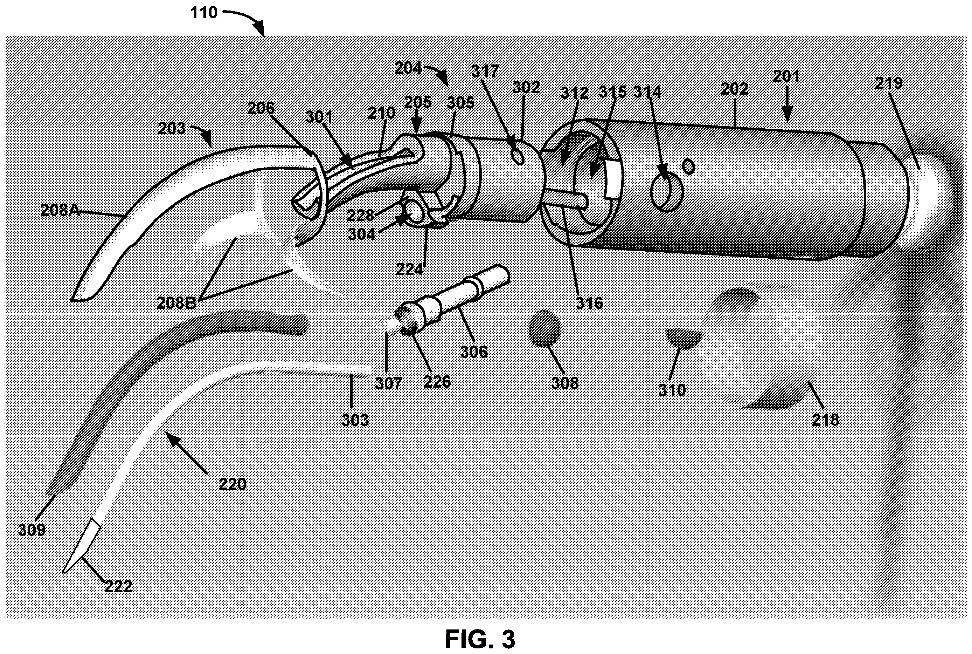

FIG. 3 is a conceptual diagram illustrating an exploded view of the example IMD 110 of FIGS. 2A-2B. As illustrated in FIG. 3, IMD 110 includes fixation component 203, EIA 204, and body 201. As illustrated in FIG. 3 and described previously with reference to FIGS. 2A-2B, fixation component 203 can include base 206 and a plurality of tines 208 (e.g., penetrating tine 208A, support tines 208B).

EIA 204 may include cap 305, elongated body 205 extending distally from cap 305, and proximal portion 302 extending proximally from cap 305. Cap 305 may be configured to be secured within body 201 (e.g., within groove 312) when IMD 110 is assembled. Cap 305 may hemodynamically isolate electronics circuitry disposed within inner chamber 315 of body 201 from blood or other bodily fluids of the patient. Cap 305 may have a larger outer diameter than elongated body 205 or proximal portion 302. First elongated structure 210 and second elongated structure 224 may extend from a distal end of cap 305. Cap 305 may include a plurality of openings (not shown) sized to allow passage of feedthrough assemblies (e.g., feedthrough assembly 303, feedthrough assembly 306) into body 201. Proximal portion 302 may be configured to be at least partially inserted into inner chamber 315. Proximal portions 316 of the feedthrough assemblies may extend proximally from proximal portion 302 and into inner chamber 315 to connect to electronic circuitry (not pictured). The plurality of openings may be connected to cannula 301 and recess 304 of first elongated structure 210 and second elongated structure 224, respectively. In some examples, proximal portion 302 may include opening(s) 317. Opening(s) 317 may be configured to accept a pin or the like, e.g., to affix EIA 204 to body 201.

Leadlet 220 may include insulative sleeve 309 and feedthrough assembly 303. As illustrated in FIG. 3, feedthrough assembly 303 may be disposed within insulative sleeve 309. When assembled, leadlet 220 may extend from distal portion 216 and may be disposed within cannula 301. Cannula 301 may be defined by first elongated structure 210 of elongated body 205 of EIA 204. Cannula 301 may be curved, e.g., along the curvature of penetrating tine 208A. In some examples, as illustrated in FIG. 3, cannula 301 may be partially open and expose leadlet 220. In other examples, cannula 301 may be fully enclosed. First electrode 222 may be exposed at a distal end of leadlet 220. In some examples, first electrode 222 may extend along the entire length of leadlet 220 (e.g., along length of feedthrough assembly 303). In other examples, as illustrated in FIG. 3, first electrode 222 may be disposed on the distal end of leadlet 220 and be connected to electronic circuitry within inner chamber 315 via feedthrough assembly 303.

Second electrode 226 may be disposed on a distal end of feedthrough assembly 306. Feedthrough assembly 306 may be disposed within recess 304 within second elongated structure 224 and be connected to electronic circuitry within inner chamber 315. In some examples, as illustrated in FIG. 3, feedthrough assembly 306 includes a therapeutic delivery module 307 (also referred to as "therapeutic substance dispensing device 307") disposed at a distal end of feedthrough assembly 306. Therapeutic delivery module 307 may deliver a therapeutic (e.g., a steroid) to target implant site 102 to reduce inflammation that may be caused by contact with second electrode 226. In some examples, therapeutic delivery module 307 may be a monolithic controlled release device (MCRD). In other examples, therapeutic delivery module 307 may include a telescoping cylindrical structure disposed within a recess defined in, or by, feedthrough assembly 306 and second electrode 226. The telescoping therapeutic delivery module 307 may be configured to be advanced out of and/or retracted into feedthrough assembly 306.

Housing 202 may define body 201 and one or more features disposed on body 201 (e.g., groove 312, opening(s) 314, or the like). As illustrated in FIG. 3, reference electrode 218 may be a conductive band removably secured to the outer surface of housing 202, e.g., via pin 310. A portion of the outer surface of housing 202 may have a smaller outer diameter such that when reference electrode 218 is secured to housing 202, reference electrode 218 is flush with the outer surface of housing 202. Opening(s) 314 may be configured to accept pin 308 and may be aligned with opening(s) 317 on EIA 204 to allow pin 308 to enter opening(s) 314 and opening(s) 317 and secure EIA 204 to housing 202. EIA 204 may be removed from housing 202 by removing pin 308 from opening(s) 314 and opening(s) 317.

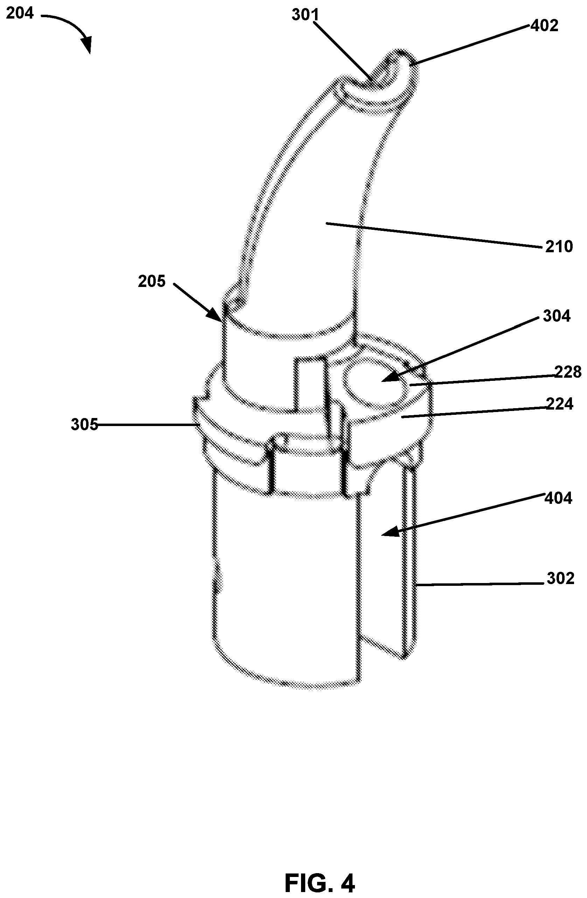

FIG. 4 is a conceptual diagram illustrating EIA 204 of FIGS. 2A-2B. As illustrated in FIG. 4, cannula 301 and recess 304 may be disposed on a same reference plane (e.g., along longitudinal axis 212). In other examples, cannula 301 and recess 304 may be disposed on different reference planes and/or not along longitudinal axis 212. Proximal portion 302 of EIA 204 may define channel 404 extending proximally from cap 305 to a proximal end of proximal portion 302. Channel 404 may be configured to retain proximal portions 316 of feedthrough assemblies (e.g., feedthrough assembly 303, feedthrough assembly 306) disposed within EIA 204.

Cannula 301 may taper to distal end 402 of cannula 301. In some examples, as illustrated in FIG. 4, distal end 402 may be chamfered, e.g., to prevent further puncture and/or penetration of the tissue by distal end 402. The outer surface of distal end 402 may have a smaller radius of curvature than a proximal end of first elongated structure 210. In other examples, the outer surface of distal end 402 may have a same radius of curvature as the proximal end of first elongated structure 210.

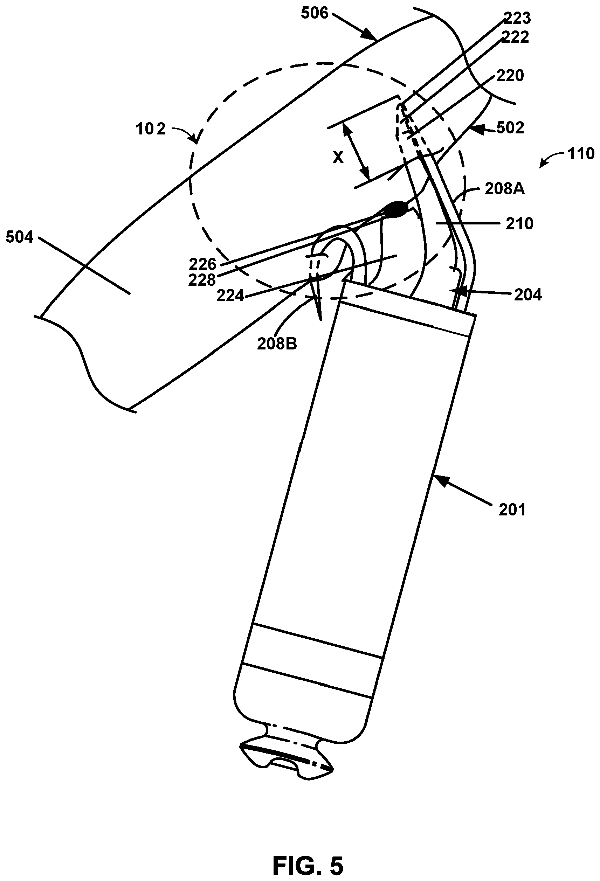

FIG. 5 is a conceptual diagram illustrating IMD 110 implanted at target implant site 102. Tines 208 may define a deformable preset curvature configured to position leadlet 220, electrode 222, and electrode 226 at selected tissue of a target implant site 102. Target implant site 102 includes a portion the right atrial RA wall, such as the atrioventricular septum having an atrial surface 502 and a ventricular surface 506. In some examples, the target implant site may include the triangle of Koch or other locations inside heart 104, such as at least one of an interatrial septum or an interventricular septum. In other examples, target implant site 102 may include other tissues within a body of a patient. When deployed at target implant site 102, tines 208 may have a deployment stiffness that enables a respective tine 208 (e.g., penetrator tine 208A, support tine 208B, etc.) to penetrate selected tissue at target implant site 102. For example, IMD 110 may be secured at target implant site 102 by tines 208 of fixation component 203 penetrating through the myocardium, such as a layer of pectinate muscle. Tines 208 may be configured for spring-loaded release upon deployment out through distal opening 108 of delivery tool 106 (FIG. 1), such that free distal ends of tines 208 (e.g., free distal end 223 of penetrator tine 208A) penetrates the myocardium. It should be noted that alternate suitable implant sites for fixation component 203 can be along any suitable surface of the heart or other tissue within a body of a patient. By controlling the deployment stiffness, tines 208 may have improved tissue fixation, including control of a depth of penetration and an amount of tissue engagement in a lateral direction.

EIA 204 may operate in conjunction with tines 208. For example, EIA 204 (e.g., second elongated structure 224 of EIA 204) may be configured to control a depth X of tissue penetration of penetrator tine 208A. Depth X may include the distance between the incisive distal tip of penetrator tine 208A and the distal end 228 of second elongated structure 224, and/or a length of the distal section of penetrator tine 208A, leadlet 220, and/or first elongated structure 210 that penetrates the tissue. In some examples, depth X of tissue penetration of penetrator tine 208A may be within a range from about 1 millimeter (mm) to about 20 mm, such as within a range from about 4 mm to about 10 mm. EIA 204 may control a depth of tissue penetration by contacting the tissue and resisting further penetration by penetrator tine 208A. In such an example, depth X of tissue penetration of penetrator tine 208A may be within a range from about 4 millimeters to about 10 millimeters.

IMD 110 may be implanted at target implant site 102 such that first electrode 222 and second electrode 226 are in contact with the selected tissue. When positioned in such a manner, first electrode 222 of leadlet 220 and second electrode 226 may function in conjunction for bipolar pacing and sensing, and/or first electrode 222 and second electrode 226 may each separately function with return electrode 218 for pacing and sensing of different selected tissues. For example, first electrode 222 of leadlet 220 may pace and/or sense ventricular tissue, and second electrode 226 may pace and/or sense atrial tissue.

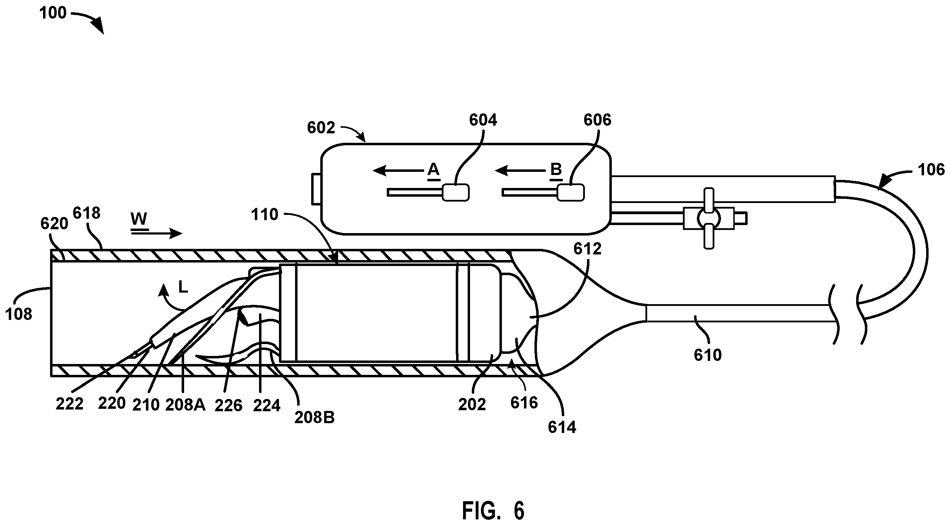

FIG. 6 is a conceptual diagram illustrating a partial cut-away section of an example medical device system 100 including a delivery tool 106 and an IMD 110. For purposes of illustration, the distal end of delivery tool 106 is enlarged relative to handle 602. Additionally, although medical device system 100 is described in reference to fixation component 203 described in reference to FIGS. 2A-5, in other examples, medical device system 100 may include other fixation components.

During use, IMD 110 may be loaded into delivery tool 106 for deployment to a target implant site (e.g., target implant site 102). Delivery tool 106 may include a handle 602, an elongate outer member 610, and an elongate inner member 612 that extends within lumen 616 of outer member 610. Outer member 610 may include a distal end 614 configured to engage IMD 110 by abutting proximal portion 214 of body 201 (e.g., as shown in the cut-away section). An entirety of IMD 110 may be loaded within tubular sidewall 618 that defines a distal portion of outer member lumen 610, for example, having been loaded therein by pulling IMD 110, with body proximal portion 214 leading, in through lumen distal opening 108. In some examples, an inner surface 620 of tubular sidewall 618 may engage tines 208 of fixation component 203 as IMD 110 is loaded into lumen 616 to deform tines 208 (per arrow L) and then to hold each tine of tines 208 of the loaded IMD 110 in a deformed configuration, e.g., a spring-loaded configuration.

Handle 602 may be configured to control movement of delivery tool 106 and/or deployment of IMD 110. The clinician may position medical device system 100 by advancing delivery tool 106 through vasculature of the patient, for example, from a femoral or jugular venous access site and through the IVC or SVC (FIG. 1). Delivery tool 106 may include articulating features to facilitate the navigation of the distal portion of delivery tool 106. For example, inner member 612 of delivery tool 106 may include a pull wire assembly (not shown) integrated therein and being coupled to another control member 604 of handle 602 that, when moved per arrow A, causes inner member 612 and outer member 610 to bend along distal portions thereof.

In some examples, a proximal end of outer member 610 may be coupled to a control member 606 of handle 602 such that an entirety of outer member 610 is movable with respect to inner member 612, via control member 606. For example, after positioning medical device system 100 at selected tissue in proximity to a target implant site 102 (FIG. 1), a clinician may retract outer member 610, per arrow W, relative to IMD 110 and inner member 612, thereby release the spring loading fixation component 203 to deploy IMD 110 out through distal opening 108 such that tines 208 engage with the selected tissue to secure IMD 110 at the implant site. Additionally, or alternatively, delivery tool 106 may be configured so that a clinician can advance inner member 612 relative to outer member 610 to push IMD 110 out through distal opening 108 for deployment. A length of outer member 610, between handle 602 and distal opening 108 may be between about 100 cm and about 120 cm.

Penetrator tine 208A may be configured to penetrate or cut a selected tissue to form a puncture. EIA 204 may be configured to protect leadlet 220 during deployment. In some examples, where first electrode 222 is molded into EIA 204, EIA 204 may protect first electrode 222 and second electrode 226 during deployment. In some examples, during deployment from delivery tool 106, penetrator tine 208A may initially penetrate selected tissue of atrial surface 502 to form a puncture. Also, EIA 204 (e.g., cannula 301 of first elongated structure 210) may urge leadlet 220 toward penetrator tine 208A, thereby guiding first elongated structure 210 and leadlet 220 into the puncture. For example, first elongated structure 210 may be configured to urge leadlet 220 toward penetrator tine 208A by applying a force to leadlet 220 in the direction of penetrator tine 208A during and/or after deployment. By urging leadlet 220 toward penetrator tine 208A, EIA 204 may reduce undesired displacement of leadlet 220 (e.g., relative to penetrator tine 208A) and/or reduce mechanical damage to leadlet 220 (e.g., as leadlet 220 passes out of delivery tool 106). In other examples, where first electrode 222 is molded into first elongated structure 210, first elongated structure 210 may be shaped to place first electrode 222 near distal end 223 of penetrator tine 208A, e.g., to guide first electrode 222 and first elongated structure 210 into the puncture.

As fixation component 203 is further deployed, penetrator tine 208A may return from the deployed configuration, for example, as illustrated in FIG. 3. Upon the penetrator tine 208A reaching the selected depth of tissue penetration, EIA 204 may begin to contact the tissue and resist further tissue penetration. When in the deployed configuration, leadlet 220 may extend in a distal direction between penetrator tine 208A and first elongated structure 210. In this way, leadlet 220 may extend from distal portion 216 of body 201 of IMD 110 to reach a selected target tissue, such as tissue near ventricular surface 506. In a similar fashion, second electrode 226 may be urged against tissue at atrial surface 502 by EIA 204, and optionally penetrator tine 208A and support tines 208B. In this way, IMD 110 may be configured to penetrate the atrioventricular septum to enable pacing and/or sensing at ventricular surface 506 via first electrode 222 and pacing and/or sensing at atrial surface 502 via second electrode 226.

After deployment at target implant site 102, in some examples, a deflection stiffness of tines 208 may enable a clinician to confirm adequate fixation of tines 208 into tissue of a patient. For example, a pull test or tug test may be performed under fluoroscopy to confirm that tines 208 have engaged the tissue and thereby confirm adequacy of implantation of IMD 110. The pull test or tug test may include the clinician pulling or tugging on the deployed IMD 110 and observing movement of tines 208 to determine if tines 208 are engaged in tissue, e.g., tines 208 that are embedded in tissue deflect or bend as deployed IMD 110 is pulled or tugged. By controlling the deflection stiffness, tines 208 may have an improved flexibility that enables a clinician to confirm tissue engagement more easily.

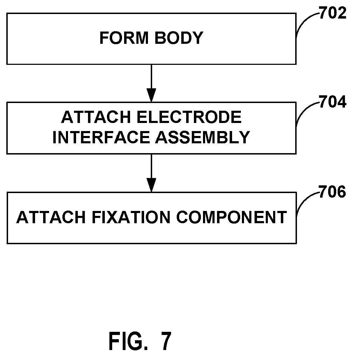

IMD 110 may be manufactured using any suitable technique. FIG. 7 is a flow diagram illustrating an example method of manufacturing IMD 110. Although the technique illustrated in FIG. 7 is described in reference to IMD 110 illustrated in reference to FIGS. 2A-5, the technique may be used to manufacture other IMDs, such as IMDs with other fixation components

A manufacturer may form body 201 extending from proximal portion 214 to distal portion 216 along longitudinal axis 212 (702). In some examples, forming body 201 may include shaping a biocompatible and biostable metal such as titanium into a hermetically sealed housing 202. The hermetically sealed housing 202 may then define body 201 and inner chamber 315. In some examples., shaping housing 202 may include forming one or more features defined by housing 202 including, but are not limited to, groove 312, opening(s) 314, or retrieval structure 219. Forming body 201 may include a pre-processing or post-processing step, such as applying a nonconductive coating and defining a return electrode 218 as an uncoated portion of body 201. In some examples, return electrode 218 may be separately manufactured and attached to body 201, e.g., to a portion of the proximal portion 214 of body 201 with a smaller outer diameter. In some examples, body 201 may be dimensioned so that an outer diameter of IMD 110 (e.g., outer diameter of body 201) may be between about 10 French (Fr) and about 30 Fr, such as about 20 Fr.

The manufacturer may attach EIA 204 to distal portion 216 of body 201 (704). EIA 204 may be attached using one or more known techniques, such as an adhesive, fastener, screw, weld, pin, and the like. EIA 204 may include proximal portion 302, cap 305, and elongated body 205 extending distally from cap 305. EIA 204 may be attached to distal portion 216 of body 201 such that elongated body 205, including first elongated structure 210 and second elongated structure 224, extends distally from distal portion 216 of body 201 along longitudinal axis 212. The manufacturer may attach EIA 204 to body 201 by inserting proximal portion 302 into body 201 and securing proximal portion 302 to housing 202 defining body 201 using one or more of the known techniques.

In some cases, EIA 204 may be integrally formed and then attached to body 201. For example, EIA 204 may be integrally formed from a polymer by one or more of molding, over-molding, additive manufacturing such as three-dimensional printing, and/or subtractive manufacturing such as machining or etching. In some examples, EIA 204 is formed to include first elongated structure 210 extending from cap 305. First elongated structure 210 may be formed to define cannula 301 extending the length of first elongated structure to distal end 402. First elongated structure 210 may be formed to partially, fully, or not encapsulate cannula 301. EIA 204 may be formed as a single component. In other examples, components of EIA 204 (e.g., elongated body 205, first elongated structure 210, second elongated structure 224) may be formed separately and assembled to form EIA 204.

In some examples, EIA 204 is formed to define recess 304 in second elongated structure 224 through which second electrode 226 and feedthrough assembly 306 may extend. Distal end 228 of second elongated structure 224 may be configured to contact a tissue to control a depth of tissue penetration of penetrator tine 208A. In other examples, a material forming EIA 204 may be over-molded around first electrode 222, second electrode 226, and corresponding feedthrough assemblies 303 and 306 as a part of the process of forming EIA 204. In such examples, first elongated structure 210 may be shaped to place first electrode 222 at the depth of tissue penetration of penetrator tine 208A, e.g., without the use of leadlet 220.

The manufacturer may create texturing on EIA 204 (e.g., on the outer surface of first elongated structure 210). The manufacturer may create the texturing using one or more known techniques including, but is not limited to, not polishing or reducing polishing on the textured portion of EIA 204, laser etching the textured portion of EIA 204, or etching the texture portion of EIA 204 using one or more other etching techniques.

The manufacturer may attach fixation component 203 to body 201 of IMD 110 (706). Fixation component 203 may be attached to body 201 using one or more known techniques, such as an adhesive, fastener, screw, weld, and the like. In some examples, fixation component 203 may be formed and then attached to body 201. For example, fixation component 203 may be formed to include base 206 and tines 208.

Forming base 206 may include forming a base having a proximal end and a distal end aligned along the longitudinal axis. The proximal end of base 206 may be attached to distal portion 216 of body 201 or to cap 305 of EIA 204. Forming base 206 may include cutting a tube, such as a metal tube, a nickel titanium alloy tube, or a stainless-steel tube, to define base 206. Forming base 206 may include pre-processing or post-processing steps, such as abrading, coating, heat treating, or polishing a substrate defining base 206.

Forming tines 208 may include extending tines 208 from base 206 and spacing tines 208 apart from one another. Tines 208 may include penetrator tine 208A. Penetrator tine 208A may include a proximal section attached to and extending from distal portion 216 of body 201 in a first direction. Penetrator tine 208A may further include a curved section defining a deformable preset curvature of penetrator tine 208A and extending in a second direction laterally from the proximal section of penetrator tine 208A and traversing longitudinal axis 212. Penetrator tine 208A may further include a distal section extending from the curved section of penetrator tine 208A and terminating in incisive distal end 223. Incisive distal end 223 may be configured to penetrate a tissue (e.g., at target implant site 102) to affix IMD 110 to the tissue.

In some examples, base 206 and tines 208 may be integrally formed. For example, base 206 and tines 208 may be integrally formed from a tube, such as a metal tube, a nickel titanium alloy tube, or a stainless-steel tube. In some examples, forming base 206 and tines 208 from a single tube may include removing material from the single tube to define base 206 and tines 208. In some examples, removing material from the single tube may include one or more of machining, chemical etching, laser etching, stamping, or water cutting. In some examples, forming tines 208 may include forming one or more tapers on one or more tines of tines 208. For example, forming one or more tapers may include any other above techniques to remove material from the single tube. In some examples, one or more tapers may be formed while removing material form the single tube.

In some examples, forming tines 208 may include bending each tine of tines 208 to define one or more curved section. In some examples, each curve and/or each tine of tines 208 may be bent individually or bend simultaneously, e.g., by use of a jig configured to bend one or more curves on one or more of tines 208. After bending (and holding in the bent configuration) tines 208, forming tines 208 also may include heat treating the bent tines 208 to cause tines 208 to hold the bent configuration. For example, heat treating the bent tines 208 may cause a microstructure of the material of tines 208 to assume a configuration such that a resting state of tines 208 (e.g., without application of an external force) is the bent configuration.

In some examples, forming tines 208 also may include sharpening distal ends of tines 208. For example, forming penetrator tine 208A may include laser etching distal end 223 of penetrator tine 208A to define an infinitely or near infinitely sharp incisive edge (within the tolerances of common manufacturing processes).

In some examples, forming tines 208 also may include forming one or more cutouts, engravings, embossing, or other variations in the thickness of tines 208. For example, cutouts, engravings, embossing, or other variations in the thickness of tines 208 may be formed by laser etching or chemical etching.

Various examples of the disclosure have been described. Any combination of the described systems, operations, or functions is contemplated. These and other examples are within the scope of the following claims.

## Claims

1. An implantable medical device (IMD), comprising:
a device body (201) extending from a proximal portion (214) to a distal portion (216) along a longitudinal axis (212);
a fixation component (203) comprising a penetrator tine (208A) extending away from the distal portion of the device body and configured to penetrate a tissue; and
an electrode interface assembly (204) comprising:
a leadlet (220) extending away from the distal portion of the device body;
an elongated body (205) extending away from the distal portion of the device body, the elongated body defining a recess (304) and a groove (301), wherein the groove is configured to receive the leadlet; and
an electrode (226) extending from the elongated body (205) and disposed within the recess, wherein the electrode is configured to form an electrical circuit with the tissue, and wherein the elongated body is configured to place the electrode in contact with the tissue.

2. The IMD of claim 1, wherein the electrode comprises a second electrode (226) and the electrical circuit comprises a second electrical circuit, and wherein the leadlet further comprises:
an elongated member extending away from the distal portion (216) of the device body (201) to an incisive distal end (223) of the penetrator tine (208A); and
a first electrode (222) disposed on the elongated member, the first electrode being configured to form a first electrical circuit with the tissue.

3. The IMD of claim 2, wherein, when the IMD is implanted within an atrium of a heart of a patient, the first electrode (222) is configured to deliver stimulation therapy to or sense cardiac signals from a ventricle of the heart, and the second electrode is configured to deliver stimulation therapy to or sense cardiac signals from the atrium.

4. The IMD of any of claims 2 and 3, wherein each electrode of the first electrode (222) and the second electrode (226) is connected, via a corresponding feedthrough assembly (303, 306) of a plurality of feedthrough assemblies, to electronic circuitry disposed within the device body of the IMD.

5. The IMD of claim 4, wherein the plurality of feedthrough assemblies are disposed on a same reference plane parallel to the longitudinal axis (212).

6. The IMD of any of claims 4 and 5, wherein each feedthrough assembly of the plurality of feedthrough assemblies are electrically isolated from any other feedthrough assembly of the plurality of feedthrough assemblies.

7. The IMD of any of claims 4-6, wherein one feedthrough assembly of the plurality of feedthrough assemblies is concentric with the device body (201).

8. The IMD of any of claims 4-6, wherein each feedthrough assembly of the plurality of feedthrough assemblies is equidistant from a radial center of the device body (201).

9. The IMD of any of claims 1-8, further comprising a return electrode (218) disposed on the device body.

10. The IMD of any of claims 1-9, wherein the electrode further comprises a therapeutic substance dispensing device (307) disposed within an aperture defined by the electrode.

11. The IMD of any of claims 1-10, wherein the therapeutic substance dispensing device (307) comprises a monolithic controlled release device.

12. The IMD of claim 11, wherein the therapeutic substance dispensing device (307) is configured to extend away from a corresponding feedthrough assembly of the electrode and towards a distal end of the electrode.

13. The IMD of any of claims 1-12, wherein the elongated body (205) of the electrode interface assembly (204) comprises a texture disposed at least partially around an outer surface of the elongated body (205), and wherein the texture is configured to increase adhesion between the outer surface of the elongated body and the tissue.

14. The IMD of any of claims 1-13, wherein a shape of the elongated body (205) is configured to control a depth of penetration of the penetrating tine (208A) into the tissue.

15. The IMD of any of claims 1-14, wherein the fixation component (203) further comprises one or more support tines (208B) extending from the distal portion (216) of the device body, wherein when the one or more support tines are in a deployed configuration, the one or more support tines are configured to affix to the tissue.

## Patentansprüche

1. Implantierbare medizinische Vorrichtung (IMD), umfassend:
einen Vorrichtungskörper (201), der sich von einem proximalen Abschnitt (214) zu einem distalen Abschnitt (216) entlang einer Längsachse (212) erstreckt;
eine Befestigungskomponente (203), umfassend einen Penetratorzinken (208A), der sich von dem distalen Abschnitt des Vorrichtungskörpers weg erstreckt und dazu ausgelegt ist, in ein Gewebe einzudringen; und
eine Elektrodenschnittstellenanordnung (204), umfassend:
ein Leadlet (220), das sich von dem distalen Abschnitt des Vorrichtungskörpers weg erstreckt;
einen länglichen Körper (205), der sich von dem distalen Abschnitt des Vorrichtungskörpers weg erstreckt, wobei der längliche Körper eine Aussparung (304) und eine Nut (301) definiert, wobei die Nut dazu ausgelegt ist, das Leadlet aufzunehmen; und
eine Elektrode (226), die sich von dem länglichen Körper (205) erstreckt und innerhalb der Aussparung angeordnet ist, wobei die Elektrode dazu ausgelegt ist, einen elektrischen Schaltkreis mit dem Gewebe zu bilden, und
wobei der längliche Körper dazu ausgelegt ist, die Elektrode in Kontakt mit dem Gewebe zu bringen.

2. IMD nach Anspruch 1, wobei die Elektrode eine zweite Elektrode (226) umfasst und der elektrische Schaltkreis einen zweiten elektrischen Schaltkreis umfasst und wobei das Leadlet ferner dies umfasst:
ein längliches Element, das sich von dem distalen Abschnitt (216) des Vorrichtungskörpers (201) weg zu einem scharfen distalen Ende (223) des Penetratorzinkens (208A) erstreckt; und
eine erste Elektrode (222), die an dem länglichen Element angeordnet ist, wobei die erste Elektrode dazu ausgelegt ist, einen ersten elektrischen Schaltkreis mit dem Gewebe zu bilden.

3. IMD nach Anspruch 2, wobei, wenn die IMD in einem Atrium eines Herzens eines Patienten implantiert ist, die erste Elektrode (222) dazu ausgelegt ist, eine Stimulationstherapie an ein Ventrikel des Herzens abzugeben oder Herzsignale von diesem zu erfassen, und die zweite Elektrode dazu ausgelegt ist, eine Stimulationstherapie an das Atrium abzugeben oder Herzsignale von diesem zu erfassen.

4. IMD nach einem der Ansprüche 2 und 3, wobei jede Elektrode der ersten Elektrode (222) und der zweiten Elektrode (226) über eine entsprechende Durchführungsanordnung (303, 306) von mehreren Durchführungsanordnungen mit elektronischen Schaltungen verbunden ist, die in dem Vorrichtungskörper der IMD angeordnet sind.

5. IMD nach Anspruch 4, wobei die mehreren Durchführungsanordnungen auf derselben Referenzebene parallel zu der Längsachse (212) angeordnet sind.

6. IMD nach einem der Ansprüche 4 und 5, wobei jede Durchführungsanordnung der mehreren Durchführungsanordnungen von allen anderen Durchführungsanordnungen der mehreren Durchführungsanordnungen elektrisch isoliert ist.

7. IMD nach einem der Ansprüche 4-6, wobei eine Durchführungsanordnung der mehreren Durchführungsanordnungen konzentrisch mit dem Vorrichtungskörper (201) angeordnet ist.

8. IMD nach einem der Ansprüche 4-6, wobei jede Durchführungsanordnung der mehreren Durchführungsanordnungen gleich weit von einer radialen Mitte des Vorrichtungskörpers (201) entfernt ist.

9. IMD nach einem der Ansprüche 1-8, ferner umfassend eine Gegenelektrode (218), die an dem Vorrichtungskörper angeordnet ist.

10. IMD nach einem der Ansprüche 1-9, wobei die Elektrode ferner eine Abgabevorrichtung für therapeutische Substanzen (307) umfasst, die in einer durch die Elektrode definierten Öffnung angeordnet ist.

11. IMD nach einem der Ansprüche 1-10, wobei die Abgabevorrichtung für therapeutische Substanzen (307) eine monolithische gesteuerte Freigabevorrichtung umfasst.

12. IMD nach Anspruch 11, wobei die Abgabevorrichtung für therapeutische Substanzen (307) dazu ausgelegt ist, sich von einer entsprechenden Durchführungsanordnung der Elektrode weg und in Richtung eines distalen Endes der Elektrode zu erstrecken.

13. IMD nach einem der Ansprüche 1-12, wobei der längliche Körper (205) der Elektrodenschnittstellenanordnung (204) eine Textur umfasst, die wenigstens teilweise um eine Außenfläche des länglichen Körpers (205) herum angeordnet ist, und wobei die Textur dazu ausgelegt ist, die Haftung zwischen der Außenfläche des länglichen Körpers und dem Gewebe zu erhöhen.

14. IMD nach einem der Ansprüche 1-13, wobei eine Form des länglichen Körpers (205) dazu ausgelegt ist, eine Eindringtiefe des Penetratorzinkens (208A) in das Gewebe zu steuern.

15. IMD nach einem der Ansprüche 1-14, wobei die Befestigungskomponente (203) ferner einen oder mehrere Stützzinken (208B) umfasst, die sich von dem distalen Abschnitt (216) des Vorrichtungskörpers erstrecken, wobei, wenn sich die ein oder mehreren Stützzinken in einer Einsatzkonfiguration befinden, die ein oder mehreren Stützzinken dazu ausgelegt sind, am Gewebe fixiert zu werden.

## Revendications

1. Dispositif médical implantable (IMD), comprenant :
un corps de dispositif (201) s'étendant d'une partie proximale (214) à une partie distale (216) le long d'un axe longitudinal (212) ;
un composant de fixation (203) comprenant une dent pénétrante (208A) s'étendant à l'écart de la partie distale du corps de dispositif et configurée pour pénétrer dans un tissu ; et
un ensemble interface d'électrodes (204) comprenant :
un petit conducteur (220) s'étendant à l'écart de la partie distale du corps du dispositif ;
un corps allongé (205) s'étendant à l'écart de la partie distale du corps du dispositif, le corps allongé définissant un évidement (304) et une rainure (301), la rainure étant configurée pour recevoir le petit conducteur ; et
une électrode (226) s'étendant à partir du corps allongé (205) et disposée à l'intérieur de l'évidement, l'électrode étant configurée pour former un circuit électrique avec le tissu, et le corps allongé étant configuré pour placer l'électrode en contact avec le tissu.

2. IMD selon la revendication 1, dans lequel l'électrode comprend une seconde électrode (226) et le circuit électrique comprend un second circuit électrique, et dans lequel le petit conducteur comprend en outre :
un élément allongé s'étendant à l'écart de la partie distale (216) du corps de dispositif (201) jusqu'à une extrémité distale incisive (223) de la dent pénétrante (208A) ; et
une première électrode (222) disposée sur l'élément allongé, la première électrode étant configurée pour former un premier circuit électrique avec le tissu.

3. IMD selon la revendication 2, dans lequel, lorsque l'IMD est implanté dans une oreillette du cœur d'un patient, la première électrode (222) est configurée pour administrer une thérapie de stimulation à un ventricule du cœur ou détecter des signaux cardiaques provenant de celui-ci, et la seconde électrode est configurée pour administrer une thérapie de stimulation à l'oreillette ou détecter des signaux cardiaques provenant de celle-ci.

4. IMD selon l'une quelconque des revendications 2 et 3, dans lequel chaque électrode de la première électrode (222) et de la seconde électrode (226) est connectée, par le biais d'un ensemble traversée (303, 306) correspondant d'une pluralité d'ensembles traversée, à des circuits électroniques disposés à l'intérieur du corps de dispositif de l'IMD.

5. IMD selon la revendication 4, dans lequel la pluralité d'ensembles traversée est disposée sur un même plan de référence parallèle à l'axe longitudinal (212).

6. IMD selon l'une quelconque des revendications 4 et 5, dans lequel chaque ensemble traversée de la pluralité d'ensembles traversée est isolé électriquement de tout autre ensemble traversée de la pluralité d'ensembles traversée.

7. IMD selon l'une quelconque des revendications 4 à 6, dans lequel un ensemble traversée de la pluralité d'ensembles traversée est concentrique au corps de dispositif (201).

8. IMD selon l'une quelconque des revendications 4 à 6, dans lequel chaque ensemble traversée de la pluralité d'ensembles traversée est équidistant d'un centre radial du corps de dispositif (201).

9. IMD selon l'une quelconque des revendications 1 à 8, comprenant en outre une électrode de retour (218) disposée sur le corps de dispositif.

10. IMD selon l'une quelconque des revendications 1 à 9, dans lequel l'électrode comprend en outre un dispositif de distribution de substance thérapeutique (307) disposé à l'intérieur d'une ouverture définie par l'électrode.

11. IMD selon l'une quelconque des revendications 1 à 10, dans lequel le dispositif de distribution de substance thérapeutique (307) comprend un dispositif monolithique à libération contrôlée.

12. IMD selon la revendication 11, dans lequel le dispositif de distribution de substance thérapeutique (307) est configuré pour s'étendre à l'écart d'un ensemble traversée correspondant de l'électrode et vers une extrémité distale de l'électrode.

13. IMD selon l'une quelconque des revendications 1 à 12, dans lequel le corps allongé (205) de l'ensemble interface d'électrode (204) comprend une texture disposée au moins partiellement autour d'une surface externe du corps allongé (205), et dans lequel la texture est configurée pour augmenter l'adhérence entre la surface externe du corps allongé et le tissu.

14. IMD selon l'une quelconque des revendications 1 à 13, dans lequel une forme du corps allongé (205) est configurée pour commander une profondeur de pénétration de la dent pénétrante (208A) dans le tissu.

15. IMD selon l'une quelconque des revendications 1 à 14, dans lequel le composant de fixation (203) comprend en outre une ou plusieurs dents de support (208B) s'étendant à partir de la partie distale (216) du corps de dispositif, dans lequel lorsque la ou les dents de support sont dans une configuration déployée, la ou les dents de support sont configurées pour être fixées au tissu.
